# EUROPEAN PATENT APPLICATION

(11) **EP 2 638 903 A1**
(43) Date of publication of application: **18.09.2013**
(21) Application number: 11839308.1
(22) Date of filing: 08.11.2011
(51) Int. Cl.: A61K 31/232, A61P 3/04, A61P 3/06, A61P 3/10, A61P 9/10, A61P 9/12

(54) **SUPPRESSOR FOR INCREASE IN BLOOD GLUCOSE LEVEL**

(30) Priority: 09.11.2010 JP 2010250870
(71) Applicant: Mochida Pharmaceutical Co., Ltd., Tokyo 160-8515 (JP); Nippon Suisan Kaisha, Ltd., Chiyoda-ku Tokyo 100-8686 (JP)
(72) Inventor: KANEHIRO, Hideo, Tokyo 160-8515 (JP); IMAI, Mariko, Tokyo 160-8515 (JP)
(74) Representative: Vossius & Partner
(86) International application number: PCT/JP2011/075723
(87) International publication number: WO 2012/063820

(57) **Abstract**

The present invention relates to an agent for inhibiting elevation in postprandial blood glucose level, which comprises an ester of EPA or DHA ester as an active ingredient, and which is to be administered between meals. The invention provides an agent for inhibiting elevation in blood glucose level that has a high level of safety and desirable characteristics such as storage stability, in particular, a means for reducing postprandial hyperglycemia.

## Description

### TECHNICAL FIELD

The present invention relates to a composition for oral ingestion having an inhibitory action on elevation in blood glucose level, in particular a composition having an inhibitory action on elevation in postprandial blood glucose level.

### BACKGROUND ART

For the purpose of treatment or prevention of diseases such as diabetes, hyperglycemia and obesity, many studies have been made on means for inhibiting elevation in blood glucose level.

Pharmaceutical preparations in which an ethyl ester of the polyunsaturated fatty acid eicosapentaenoic acid (the ester is hereinafter referred to as EPA-E) is contained as an active ingredient (which are exemplified by Epadel®) are used as pharmaceutical products for treating or preventing arteriosclerosis obliterans and hyperlipidemia. In order for EPA-E to be absorbed via the gastrointestinal tract to enter the bloodstream, secreted bile acid or an ingredient from food is required as a carrier and, therefore, administration of EPA-E immediately after meal (within 30 minutes after meal) is prescribed as a dosage regimen. Studies have also been made on the effect of an EPA-E preparation on glucose metabolism, but there have been contradictory reports on the effect of this preparation: one report showing that administration of this preparation immediately after meal has an adverse effect on glucose metabolism, and another report showing that such administration has at least no adverse effect (Non-Patent Documents 1 and 2).

Glucagon-like peptide-1 (GLP-1) is known as a peptide hormone that involves activation of glucose metabolism through regulation of insulin secretion. WO 2005/083070 A1 (Patent Document 1) reports that the G-protein coupled receptor GPR120 is distributed on the surface of human intestinal GLP-1 secreting cells and is involved in promotion of GLP-1 secretion. This document also discloses that free fatty acids such as docosahexaenoic acid (DHA) and eicosapentaenoic acid (EPA) act as a ligand for GPR120. Another report shows that in a test using mice, administration of docosahexaenoic acid (DHA) and eicosapentaenoic acid (EPA) as free fatty acids directly into the colon promotes GLP-1 secretion (Non-Patent Document 3). Yet another report shows that oral administration of a pharmaceutical preparation containing a perilla oil as an ingredient to mice that had been fasted for at least 18 hours led to an increase in a GLP-1 level (Non-Patent Document 4).

### CITATION LIST

### PATENT DOCUMENTS

[Patent Document 1] International Patent Publication No. WO 2005/083070 A1

### NON-PATENT DOCUMENTS

[Non-Patent Document 1] F. Tetsuhiro, et al., Effect of EPA administration on glucose metabolism in patients with NIDDM, Journal of Clinical and Experimental Medicine (IGAKU NO AYUMI), 1994, Vol. 169, No. 8, p. 889-890
[Non-Patent Document 2] T. Shinjo, et al., Effect of Epadel on diabetes, The Japanese Journal of Clinical and Experimental Medicine, 1993, Vol. 71, No. 10, p. 2716-2718
[Non-Patent Document 3] Morishita, et al., Usefulness of colon targeted DHA and EPA as novel diabetes medications that promote intrinsic GLP-1 secretion, Journal of Controlled Release, 2008, Vol. 132, p. 99-104
[Non-Patent Document 4] Adachi, et al., Administration of Perilla Oil Coated with Calshell Increases Glucagon-Like Peptide Secretion, Biol. Pharm. Bull., 2008, Vol. 31, No. 5, p. 1021-1023

### SUMMARY OF INVENTION

### TECHNICAL PROBLEM

Free fatty acids are known as ligands for the receptor (GPR120) involved in the promotion of GLP-1 secretion, but EPA and DHA in the form of free fatty acids are very unstable to oxidation; therefore, pharmaceutical preparations containing EPA and DHA as free fatty acids require taking measures for improving storage stability. In contrast, a method for lowering blood glucose level by orally administering an ester of EPA or DHA still remains to be known.

An object of the present invention is to provide an agent for inhibiting elevation in blood glucose level that has a high level of safety and desirable characteristics such as storage stability, in particular, a means for reducing postprandial hyperglycemia.

### SOLUTION TO PROBLEM

The present inventors made intensive studies in order to achieve the above-noted object and, as a result, found that administration of an EPA ester or a DHA ester under particular conditions has an inhibitory effect on elevation in blood glucose level. Accordingly, the inventors completed the present invention.

More specifically, according to one aspect of the present invention, the following agents for inhibiting elevation in blood glucose level set forth in (1) to (4) are provided:
(1) An agent for inhibiting elevation in blood glucose level, which comprises an eicosapentaenoic acid (EPA) ester or a docosahexaenoic acid (DHA) ester as an active ingredient, and which is to be administered between meals;
(2) The agent for inhibiting elevation in blood glucose level as set forth in (1) above, which is to be administered between 2 hours after a meal and 1 hour before the next meal;
(3) The agent for inhibiting elevation in blood glucose level as set forth in (1) or (2) above, wherein the EPA ester or the DHA ester is a triglyceride comprising EPA or DHA as a constituent fatty acid or is a C₁₋₅ alkyl ester of EPA or DHA; and
(4) The agent for inhibiting elevation in blood glucose level as set forth in any one of (1) to (3) above, comprising, as an active ingredient, a purified fish oil whose total content of EPA and DHA in all fatty acids is at least 20% by weight.

Furthermore, according to another aspect of the present invention, the following processes for inhibiting elevation in blood glucose level as set forth in (5) to (8) are provided:
(5) A process for inhibiting elevation in blood glucose level, the process comprising administering an EPA ester or a DHA ester between meals;
(6) The process for inhibiting elevation in blood glucose level as set forth in (5) above, wherein the EPA ester or the DHA ester is administered between 2 hours after a meal and 1 hour before the next meal;
(7) The process for inhibiting elevation in blood glucose level as set forth in (5) or (6) above, wherein the EPA ester or the DHA ester is a triglyceride comprising EPA or DHA as a constituent fatty acid or is a C₁₋₅ alkyl ester of EPA or DHA; and
(8) The process for inhibiting elevation in blood glucose level as set forth in any one of (5) to (7) above, the process comprising using, as an active ingredient, a purified fish oil whose total content of EPA and DHA in all fatty acids is at least 20% by weight.

### ADVANTAGEOUS EFFECTS OF INVENTION

The agent for inhibiting elevation in blood glucose level according to the present invention has an excellent inhibitory effect on elevation in blood glucose level and is particularly effective in inhibiting elevation in postprandial blood glucose level. Further, since EPA and DHA have a therapeutic and preventive effect on arteriosclerosis obliterans and hyperlipidemia, the inventive agent for inhibiting elevation in blood glucose level can be used for treatment and prevention of lifestyle-related diseases such as hypertension, hyperglycemia, and obesity.

### BRIEF DESCRIPTION OF DRAWINGS

FIG. 1 is a graph showing the change in the average blood glucose levels of 7 subjects. The vertical axis represents blood glucose level (mg/dL) and the horizontal axis time after OGTT (min).
FIG. 2 is a graph showing the AUCs in FIG. 1.
FIG. 3 is a graph showing the changes in the blood glucose levels of a subject. The vertical axis represents blood glucose level (mg/dL) and the horizontal axis time after OGTT (min).
FIG. 4 is a graph showing the changes in the blood glucose levels of a subject. The vertical axis represents blood glucose level (mg/dL) and the horizontal axis time after OGTT (min).

### DESCRIPTION OF EMBODIMENTS

The agent for inhibiting elevation in blood glucose level according to the present invention is a composition for oral ingestion that comprises an EPA ester or a DHA ester as an active ingredient. In one mode of the present invention, the agent for inhibiting elevation in blood glucose level comprises a mixture of an EPA ester and a DHA ester as an active ingredient. The EPA ester and DHA ester to be used in the present invention is not particularly limited as long as they can be used for oral ingestion. Examples of the EPA ester or the DHA ester that can be used include extracts obtained from natural oils by known methods, partially purified products thereof, and products further highly purified therefrom. Specific examples that can be used include, but are not limited to, monoglycerides, diglycerides and triglycerides each comprising EPA or DHA as a constituent fatty acid. For example, a fish oil such as sardine oil, tuna oil, saury oil, mackerel oil, horse mackerel oil, or whiting oil is treated by a known purification method such as deacidification, decolorization, deodorization, degumming, or dewaxing, and is optionally subjected to a further purification step such as solvent fractionation, urea addition, or molecular distillation, whereby a concentrated mixture of EPA and DHA esters can be prepared.

Other examples of the ingredient that can be used in the present invention include commercially available purified fish oils containing an EPA ester or a DHA ester, such as Incromega F2250, F2628, E2251, F2573, TG2162, TG2779, TG2928, TG3525 and E5015 (Croda International PLC, Yorkshire, England), and EPAX6000FA, EPAX5000TG, EPAX4510TG, EPAX2050TG, EPAX7010EE, K85TG, K85EE and K80EE (Pronova Biopharma, Lysaker, Norway).

As the EPA ester or the DHA ester according to the present invention, an EPA or DHA derivative typically produced by microorganisms can also be used.

In one mode of the present invention, a triglyceride comprising EPA or DHA as a constituent fatty acid is used. For example, in the case where the triglyceride comprising EPA or DHA as a constituent fatty acid is used as an active ingredient of the agent, a purified fish oil containing EPA and/or DHA can be incorporated in the agent. As a purified fish oil, a fish oil such as sardine oil, tuna oil, saury oil, mackerel oil, horse mackerel oil, or whiting oil can be purified for use. For the purification, a method known in the art, such as a method typically disclosed in WO 95/24459 or WO 2007/119811, can be used.

As the agent for inhibiting elevation in blood glucose level according to the present invention, a commercially available pharmaceutical preparation comprising a triglyceride comprising EPA or DHA as a constituent fatty acid can also be used. Examples of the commercially available pharmaceutical preparation include, but are not limited to, "EPA & DHA" manufactured by Mochida Pharmaceutical, "DHA/EPA" manufactured by Morishita Jintan, "IMARK", "Umi no Genki EPA" and "Umi no Genki DHA" manufactured by Nippon Suisan, "EPA & DHA + Sesamin E" manufactured by Suntory, "DHA & EPA" manufactured by Ajinomoto, "EPA GOLD" manufactured by Raffinée International, "EPA" manufactured by FANCL, and "EPA" manufactured by DHC. The purity of an EPA ester or a DHA ester is not particularly limited, but the total content of DHA and EPA in all fatty acids contained in a purified fish oil as constituent fatty acids and free fatty acids of a triglyceride and the like is, for example, at least 20% by weight, preferably at least 30% by weight, and more preferably at least 40% by weight.

In one mode of the present invention, a C₁₋₅ alkyl ester of EPA or DHA is used. As the C₁₋₅ alkyl ester of EPA or DHA, an EPA ethyl ester or a DHA ethyl ester can be preferably used. The C₁₋₅ alkyl ester of EPA or DHA can be prepared by a known method. An example of the EPA ethyl ester that can be used is a high-purity EPA ethyl ester which is used as a therapeutic agent for arteriosclerosis obliterans (ASO) and hyperlipidemia.

As the agent for inhibiting elevation in blood glucose level according to the present invention, a commercially available pharmaceutical preparation containing an EPA ester or a DHA ester can also be used. An example of the pharmaceutical preparation containing an EPA ester is a soft capsule containing a high-purity EPA ethyl ester (at least 96.5% by weight) (product name: Epadel, manufactured by Mochida Pharmaceutical). Examples of a mixture of an EPA ethyl ester and a DHA ethyl ester include, but are not limited to, therapeutic agents for hypertriglyceridemia which are commercially available in the United States, such as Lovaza (manufactured by GlaxoSmithKline; a soft capsule containing about 46% by weight of an EPA ethyl ester and about 37% by weight of a DHA ethyl ester), and Omacor (manufactured by Pronova Biopharma; a soft capsule containing about 46% by mass of an EPA ethyl ester and about 38% by mass of a DHA ethyl ester).

The amount of an ethyl ester of EPA or DHA contained in lipid components of a pharmaceutical preparation is, for example, at least 20% by weight, preferably at least 50% by weight, and more preferably at least 80% by weight as the total of DHA and EPA esters.

The agent for inhibiting elevation in blood glucose level according to the present invention is characterized in that it is to be administered between meals. For example, the inventive agent for inhibiting elevation in blood glucose level is to be administered between 2 hours after a meal and 1 hour before the next meal, preferably between 2 and 6 hours after a meal but until 1 hour before the next meal, and more preferably between 3 and 5 hours after a meal but until 1 hour before the next meal. In one mode, the inventive agent for inhibiting elevation in blood glucose level is to be consumed between 2 and 6 hours after dinner but before bedtime.

The dose of the inventive agent for inhibiting elevation in blood glucose level can be adjusted as appropriate depending on various factors such as dosage form, administration method, administration frequency per day, level of symptom, body weight, and age. The amount of active ingredients contained in the daily dose of the present invention is exemplified by 100-2,700 mg/day, preferably 200-1,800 mg/day, and more preferably 300-1,200 mg/day, as calculated for free EPA or DHA. The daily dose may be given at one time or as divided into several portions per day, depending on the need. It is preferred to take a portion of the dose three times per day: for example, once between breakfast and lunch, once between lunch and dinner, and once after dinner but before bedtime. Because of the very high safety of the active ingredient, the inventive agent for inhibiting elevation in blood glucose level is suitable for chronic administration, for example, continuous administration for at least one week, preferably for at least one month, and more preferably for at least at least 3 months.

According to the present invention, administration of an ester of EPA or DHA between meals prevents or delays absorption of EPA or DHA from the upper gastrointestinal tract into the blood due to bile acid secretion, so that the amount of free EPA or DHA reaching the lower gastrointestinal tract can be increased and that an excellent inhibitory effect on elevation in blood glucose level can be exhibited.

The dosage form of the pharmaceutical preparation is not particularly limited as long as said preparation can be orally administered, and exemplary dosage forms that can be used include, but are not limited to, tablets, capsules (hard and soft), microcapsules, granules, fine granules, powders, oral liquid preparations (e.g., emulsions, suspensions), syrups, and jellies. The dosage forms encapsulated in, for example, capsules, in particular, soft capsules or microcapsules are preferred. Each type of pharmaceutical preparation is prepared by a method known in the art using common additives such as excipient, diluent, binding agent, humectant, disintegrating agent, disintegration inhibiting agent, absorption enhancing agent, lubricating agent, solubilizing agent, buffering agent, emulsifying agent, and suspending agent. Other additives that can be used include those which are commonly used depending on the dosage form, such as stabilizing agent, preservation agent, buffering agent, isotonizing agent, chelating agent, pH adjusting agent, surfactant, coloring agent, flavor, flavoring agent, and sweetener.

Because of the very high safety of the active ingredient, the inventive agent for inhibiting elevation in blood glucose level can be used in any subjects without particular limitation; for example, the agent can be used for healthy people to alleviate glycophilia or to prevent hyperglycemia. The inventive agent for inhibiting elevation in blood glucose level can be used for purposes such as treating hyperglycemic patients who have a fasting blood glucose level of at least 126 mg/dL or a 2-hour oral glucose tolerance test (OGTT) level of at least 200 mg/dL. The inventive agent for inhibiting elevation in blood glucose level can also be preferably used for those who have a 2-hour OGTT glucose level of 140-199 mg/dL (prediabetic level) or those who have other risk factors than hyperglycemia (e.g., hypertension, obesity, and lifestyles such as alcohol ingestion and smoking). Postprandial hyperglycemia leads to arteriosclerosis and enhances the risk of developing myocardial infarction and cerebral stroke; therefore, even if the blood glucose level is at the prediabetic or non-diabetic level, it is beneficial from the viewpoint of risk prevention to reduce elevation in postprandial blood glucose level. Accordingly, the inventive agent for inhibiting elevation in blood glucose level can be used to maintain the health of healthy people.

The present invention can be used to prevent or treat diseases related to elevation in blood glucose level, such as diabetes, hyperglycemia, impaired glucose tolerance, insulin resistance, impaired fasting glycemia, diabetic neuropathy, diabetic nephropathy, diabetic retinopathy, ketoacidosis, hyperlipidemia, hypercholesterolemia, hypertriglyceridemia, dyslipidemia, hyperlipoproteinemia, metabolic syndrome, obesity, and atherosclerosis. In particular, the present invention can be used to treat or prevent diabetes, hyperglycemia, impaired glucose tolerance, impaired fasting glycemia, and diabetic complications. The diabetes to be treated or prevented preferably refers to non-insulin dependent type II diabetes. "Diabetic complications" refers to concomitant systemic or local diseases that result either directly or indirectly from diabetes (preferably non-insulin dependent type II diabetes), and can be specifically exemplified by diabetic acidosis, diabetic xanthoma, diabetic amyotrophy, diabetic ketosis, diabetic coma, diabetic gastropathy, diabetic gangrene, diabetic ulcer, diabetic complications, diabetic diarrhea, diabetic microangiopathy, diabetic glomeruloselerosis, diabetic cardiomyopathy, diabetic neuropathy, diabetic nephropathy, diabetic bullosis, diabetic cataract, diabetic dermopathy, diabetic scleredema, diabetic retinopathy, necrobiosis lipoidica diabeticorum, and diabetic bloodstream disorder.

The agent for inhibiting elevation in blood glucose level according to the present invention can be used concomitantly with other agents, in particular, other agents for inhibiting elevation in blood glucose level, or therapeutic or preventive agents for diabetes. Exemplary concomitant agents include, but are not limited to, insulin preparation (injection), fructose-1,6-bisphosphatase (FBPase) inhibitor, glucagon receptor antagonist, glucocorticoid receptor antagonist, glucokinase activator, glutamine:fructose-6-phosphatase aminotransferase (GFAT) inhibitor, glycogen phosphorylase (GP) inhibitor, glycogen synthase kinase 3 (GSK-3) inhibitor, GPR40 agonist, phosphoenolpyruvate carboxykinase (PEPCK) inhibitor, protein tyrosine phosphatase 1B (PTPase 1B) inhibitor, pyruvate dehydrogenase kinase (PDHK) inhibitor, SGLUT inhibitor, SH2 domain-containing inositol phosphatase (SHIP2) inhibitor, dipeptidyl peptidase IV (DPP-IV) inhibitor, tGLP-1 peptide analog, α-glucosidase inhibitor, insulin sensitivity enhancer, sulfonylurea receptor agonist (SU agent), short-acting insulin secretion stimulating agent (nateglinide), low molecular weight tGLP-1 receptor agonist, low molecular weight oral insulin preparation, biguanide, 11β-HSD-1 inhibitor, adiponectin receptor agonist, AMP-activated protein kinase (AMPK) activator, PPARγ receptor agonist/antagonist, and β3-adrenergic receptor agonist. Other concomitant agents include, but are not limited to, therapeutic or preventive agents for hyperlipidemia, those for obesity, those for diabetic complications, and those for hypertension. The above-mentioned concomitant agents may be administered separately from the inventive agent for inhibiting elevation in blood glucose level, or may be administered as components of a formulation comprising the inventive agent for inhibiting elevation in blood glucose level.

According to one aspect of the present invention, there is provided a pharmaceutical composition for inhibiting elevation in blood glucose level, which comprises an EPA ester or a DHA ester as an active ingredient, and which is to be administered between meals. Said pharmaceutical composition can be used to prevent or treat diseases related to elevation in blood glucose level, such as diabetes, hyperglycemia, impaired glucose tolerance, insulin resistance, impaired fasting glycemia, diabetic neuropathy, diabetic nephropathy, diabetic retinopathy, ketoacidosis, hyperlipidemia, hypercholesterolemia, hypertriglyceridemia, dyslipidemia, hyperlipoproteinemia, metabolic syndrome, obesity, and atherosclerosis.

Because having a highly safe active ingredient, the agent for inhibiting elevation in blood glucose level according to the present invention can also be used as an ingredient of a food. The suitable food for use in the present invention can be exemplified by dietary supplements, general foods, functional foods, foods for specified health uses (FOSHU), nutritional supplementary foods, foods for elderly people, or quasi drugs.

The present invention is preferably used particularly as an active ingredient of functional foods, FOSHUs, nutritional supplementary foods, or the like. Said foods are prepared in various dosage forms such as tablets, capsules (hard and soft), microcapsules, granules, fine granules, powders, oral liquid preparations (e.g., emulsions, suspensions, self-emulsifying liquid preparations), syrups, and jellies. The foods containing the inventive agent for inhibiting elevation in blood glucose level can be labeled as, for example, health food, functional food, enteral nutrition food, FOSHU, nutritional supplementary food, food for elderly people, or quasi drug, or can also be marked with health claims approved by the Ministry of Health, Labor and Welfare in Japan, e.g., FOSHU health claim, or with health claims approved by similar regulatory systems. Examples of the latter health claim include, but are not limited to, the qualified FOSHU health claim, the health claim concerning the effect on body structure or function, and the Reduction of Disease Risk FOSHU health claim. For example, the present invention can be used in such foods as those with a health claim saying that they are intended for use in inhibiting elevation in blood glucose level, for example, those with a health claim saying "For those who are concerned about postprandial blood glucose level" or "For those who began to feel concerned about blood glucose level."

### EXAMPLES

The present invention will be more specifically described below with reference to Examples, but the present invention is not limited to these Examples.

### Study on the inhibitory Effect on Elevation in Blood Glucose Level

Healthy subjects (7 in total, 4 males and 3 females; aged 43-60) who had a fasting blood glucose level of not higher than 126 mg/dL and an oral 30-min glucose tolerance test (OGTT) level of 130-200 mg/dL were studied to determine the effects of an EPA ester or a DHA ester on elevation in blood glucose level after OGTT.

The pharmaceutical preparations used in this study were the following commercially available products: "EPA & DHA" manufactured by Mochida Pharmaceutical and "DHA/EPA" manufactured by Morishita Jintan. The details of these samples are shown in Table 1.

**[Table 1]**

| Table 1 Sample pharmaceutical preparations | | |
|---|---|---|
| Sample name | Sample 1 | Sample 2 |
| | "EPA & DHA" (Mochida Pharmaceutical) | "DHA/EPA" (Morishita Jintan) |
| Form | Soft capsule | Seamless microcapsule |
| Principal ingredient | Purified fish oil | Purified fish oil |
| Other ingredients | Antioxidant (extracted vitamin E), encapsulating agent (starch, glycerol), gelling agent, pH adjusting agent | Gelatin, edible fat and oil, emulsifying agent, sorbitol, thickening agent (pectin), flavor |

| Quantity per dose | 4 capsules (1.84 g) | 1 pack (1.8 g) |
|---|---|---|
| EPA and DHA | EPA: 333 mg | EPA: 33-155 mg |
| contents per dose | DHA: 143 mg | DHA: 300 mg |
| | (in 1.2 g of lipids) | (in 1.2 g of lipids) |

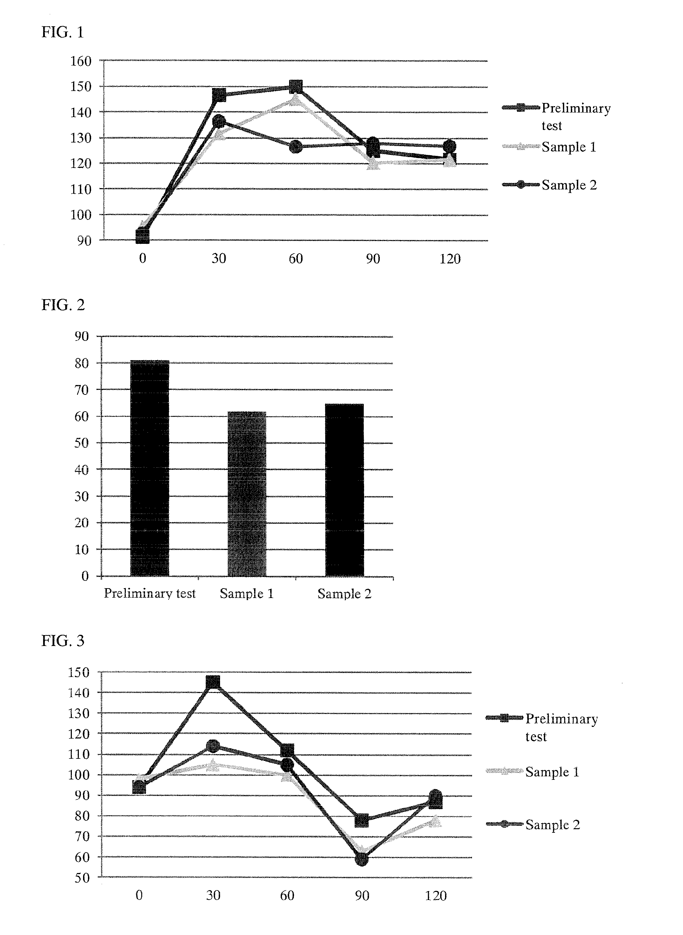

The day before the test, the subjects had a standard evening meal between 18:00 and 20:00, fasted after 20:00, and orally consumed one of the above-mentioned sample pharmaceutical preparations at 23:00. On the day of the test, they first had their blood drawn, were orally given glucose (75 g) at 9:00 (a.m.), and had their blood samples taken again after 30, 60, 90 and 120 minutes, so that the changes in their blood glucose levels were determined. The oral glucose tolerance test (OGTT) was conducted for each subject three times in total: the first run was performed without ingestion of any sample (preliminary test), the second run after ingestion of Sample 1, and the third run after ingestion of Sample 2. The runs were separated by an interval of one week. FIG. 1 shows the transitions of the average blood glucose levels of the 7 subjects. FIG. 2 shows the AUCs shown in the plot in FIG. 1. In both cases of ingestion of Samples 1 and 2, the inhibitory effect on elevation in blood glucose levels after glucose loading was found to be exhibited.

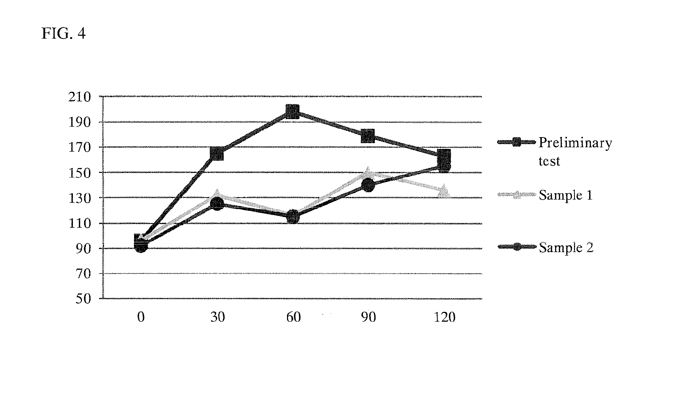

FIGs. 3 and 4 show the transitions of the blood glucose levels of two representative subjects A and B, respectively. Subject A had an evening meal (*Chirashi-zushi* [a kind of sushi with toppings scattered on a bed of rice] and salad; usual quantity) until 20:00 on the day of ingestion of Sample 1 (i.e., the day before the test) and had an evening meal (*Anago-don* [a bowl of rice with broiled conger eel], bread, *Hiyashi-chuka* [cold Chinese noodle], salad, and swiss roll; larger-than-usual quantity) until 20:00 on the day of ingestion of Sample 2 (i.e., the day before the test). Subject B had an evening meal (Sushi, salad, vegetable dish dressed with sesame sauce, Satsuma orange, and jelly; usual quantity) until 20:00 on the day of ingestion of Sample 1 (i.e., the day before the test) and had an evening meal (Sushi roll and jelly; usual quantity) until 20:00 on the day of ingestion of Sample 2 (i.e., the day before the test). Subject A had a non-diabetic 2-hour OGTT glucose level of lower than 140 mg/dL, while Subject B had a prediabetic 2-hour OGTT glucose level of 140-199 mg/dL, but both subjects were shown to experience the inhibitory effect of the ingestion of the sample pharmaceutical preparations on elevation in blood glucose levels after glucose loading.

This study demonstrated that oral ingestion of an ester of EPA or DHA between meals enables inhibition of elevation in postprandial blood glucose level caused by ingestion of the next meal (which, in this study, corresponds to the OGTT performed in the next morning).

## Claims

1. An agent for inhibiting elevation in blood glucose level, which comprises an eicosapentaenoic acid ester or a docosahexaenoic acid ester as an active ingredient, and which is to be administered between meals.

2. The agent for inhibiting elevation in blood glucose level according to Claim 1, which is to be administered between 2 hours after a meal and 1 hour before the next meal.

3. The agent for inhibiting elevation in blood glucose level according to Claim 1 or 2, wherein the eicosapentaenoic acid ester or the docosahexaenoic acid ester is a triglyceride comprising eicosapentaenoic acid or docosahexaenoic acid as a constituent fatty acid or is a C₁₋₅ alkyl ester of eicosapentaenoic acid or docosahexaenoic acid.

4. The agent for inhibiting elevation in blood glucose level according to any one of Claims 1-3, comprising, as an active ingredient, a purified fish oil whose total content of eicosapentaenoic acid and docosahexaenoic acid in all fatty acids is at least 20% by weight.
